# EUROPEAN PATENT APPLICATION

(11) **EP 4 831 287 A1**
(43) Date of publication of application: **28.10.2026**
(21) Application number: 24908277.7
(22) Date of filing: 13.12.2024
(51) Int. Cl.: C12N 15/113, C12N 9/16, C12N 9/00, C12N 15/11

(54) **NOVEL CIRCULAR RNA SYNTHESIS METHOD USING RNA LIGASE**

(30) Priority: 18.12.2023 KR 20230184409; 04.09.2024 KR 20240120267
(71) Applicant: DX&VX Co., Ltd., Seoul 08511 (KR)
(72) Inventor: KANG, Ho Young, Seoul 08377 (KR); KIM, Do Hyung, Seoul 08377 (KR); KIM, Yoon Seob, Seoul 08377 (KR); LEE, Won Gyu, Seoul 08377 (KR)
(74) Representative: Reddie & Grose LLP
(86) International application number: PCT/KR2024/096908
(87) International publication number: WO 2025/135995

(57) **Abstract**

The present invention relates to a novel circular RNA synthesis method using RNA ligase. By using a circular RNA precursor having a specific structure according to the present invention to convert a triphosphate at the 5' end into a hydroxyl group (-OH) with a phosphatase, and inducing a 2', 3'-cyclic phosphate at the 3' end by a ribozyme, and connecting them with RtcB RNA ligase, it is possible to produce/synthesize longer circular RNA with significantly higher efficiency than conventional methods.

## Description

### [TECHNICAL FIELD]

The present invention relates to a novel circular RNA synthesis method using RNA ligase.

### [BACKGROUND ART]

Circular RNA is a single-stranded RNA molecule in which a 5' end and a 3' end are connected by a covalent bond, and is known to be produced through back-splicing in various eukaryotes. Due to its structural characteristic of lacking ends, circular RNA is not easily degraded by an exonuclease and thus exists much more stably *in vivo* compared to a messenger RNA (mRNA) of a similar size. More than tens of thousands of types of circRNAs have been identified in various organisms through RNA-seq data and newly developed bioinformatics approaches, and it has been revealed that eukaryotic circular RNAs possess various molecular and cellular regulatory functions (Chen LL. The biogenesis and emerging roles of circular RNAs. Nat Rev Mol Cell Biol. 2016 Apr;17(4):205-11.). For example, circular RNA is known to be able to regulate the expression of a target gene by binding to a microRNA or by directly binding to a target protein.

Meanwhile, since mRNA has great potential in various fields such as vaccines and therapeutics, development of pharmaceuticals using the same has been active recently, but mRNA has a limitation in that it has a relatively short half-life because it is easily degraded by an exonuclease or the like *in vivo*. In order to overcome such a limitation, research is being conducted to change the terminal structure of mRNA or attach a poly(A) tail to improve stability. In a similar context, US 10,953,033 discloses circular RNA for the purpose of gene expression based on the excellent *in vivo* stability of circular RNA.

Circular RNA may be synthesized artificially *in vitro* as well as *in vivo*. As methods for *in vitro* synthesis, a "chemical method," a "method using a Group I Intron ribozyme (Ribozyme)," and a "method using an RNA ligase (ligase)" are known (Obi and Chen. The design and synthesis of circular RNAs. Methods. 196:85-103. 2021). The chemical method has disadvantages in that it is expensive and there is a limit to the size of circular RNA that may be produced. The method using a ribozyme such as Group I Intron has an advantage in that relatively excellent circularization efficiency may be achieved, but may be problematic when used as a therapeutic agent because it induces immunogenicity more strongly than the method using an RNA ligase (Liu et al., RNA circles with minimized immunogenicity as potential PKR inhibitors, Mol Cell. 82(2):420-434. 2021). In addition, the ribozyme method has disadvantages in that nicked circular RNA is produced at a high ratio during the synthesis process and it is difficult to remove the nicked circular RNA even during the purification process (Wesselhoeft et al., RNA circularization diminishes immunogenicity and may extend translation duration in vivo, Mol Cell. 74(3):508-520. 2019). In the RNA ligase method, T4 RNA ligase 1, T4 RNA ligase 2, and tRNA ligase are mainly used to induce circularization of linear RNA, and while it shows better circularization efficiency than the chemical method, it has a disadvantage of showing lower circularization efficiency than the ribozyme method. On the other hand, the RNA ligase method has advantages in that nicked circular RNA is not produced and it shows relatively low immunogenicity.

Therefore, the present inventors have completed the present invention by researching a method to significantly improve circularization efficiency, while preventing the production of nicked circular RNA and reducing the induction of immunogenicity, in the production of circular RNA for the purpose of protein expression.

### [DETAILED DESCRIPTION OF THE INVENTION]

### [TECHNICAL PROBLEM]

An object of the present invention is to provide a circular RNA precursor.

In addition, the present invention provides a recombinant nucleic acid vector for generating a circular RNA precursor.

In addition, the present invention provides a method for producing circular RNA.

Furthermore, the present invention provides a use of a circular RNA precursor.

### [TECHNICAL SOLUTION]

To achieve the above object, the present invention provides a circular RNA precursor having a structure of 5'-5' homology region-gene of interest (GOI)-3' homology region-ribozyme-3'.

In addition, the present invention provides a recombinant nucleic acid vector for generating the circular RNA precursor.

In addition, the present invention provides a method for producing circular RNA.

Furthermore, the present invention provides a use of a circular RNA precursor for producing circular RNA.

### [ADVANTAGEOUS EFFECTS]

By using a circular RNA precursor having a specific structure according to the present invention to convert a triphosphate at the 5' end into a hydroxyl group (-OH) with a phosphatase, to induce a 2', 3'-cyclic phosphate at the 3' end by a ribozyme, and to ligate them with RtcB RNA ligase, it is possible to produce/synthesize a longer circular RNA with significantly higher efficiency compared to the prior art.

### [BRIEF DESCRIPTION OF THE DRAWINGS]

FIG. 1 is a schematic diagram illustrating a method for synthesizing a circular RNA of the present invention.
FIG. 2 is a diagram showing the structure of a precursor RNA of the present invention.
FIG. 3 is a diagram showing the structure of a precursor RNA (racRNA) for producing a conventional circular RNA.
FIG. 4 is a diagram analyzing transcription efficiency according to a 5' terminal start sequence of a 5' homology region of a precursor rpcRNA template structure in a transcription step.
FIG. 5 is a diagram showing a circular RNA synthesis process of rpcRNA and racRNA.
FIG. 6 is a diagram showing the results of circular RNA synthesis and the synthesis efficiency obtained by the process of FIG. 4.
FIG. 7 is a diagram confirming circular RNA synthesis efficiency according to a concentration of GTP during circular RNA synthesis using E. coli-derived RtcB RNA ligase.
FIG. 8 is a diagram confirming circular RNA synthesis efficiency according to a concentration of Mn (concentration of GTP: 20 nM) during circular RNA synthesis using E. coli-derived RtcB RNA ligase.
FIG. 9 is a diagram showing the circular RNA synthesis efficiency according to the concentrations of GTP and Mn during circular RNA synthesis using E. coli-derived RtcB RNA ligase.
FIG. 10 is a diagram showing the circular RNA synthesis efficiency according to ligation reaction time during circular RNA synthesis using E. coli-derived RtcB RNA ligase.
FIG. 11 is a diagram confirming circular RNA synthesis efficiency according to a ligation reaction temperature during circular RNA synthesis using E. coli-derived RtcB RNA ligase.
FIG. 12 is a diagram verifying circular RNA synthesis by treating circular RNA synthesized using E. coli-derived RtcB RNA ligase with RNase R.
FIG. 13 is a diagram confirming circular RNA synthesis efficiency according to a concentration of GTP during circular RNA synthesis using human-derived RtcB RNA ligase.
FIG. 14 is a graph showing circular RNA synthesis efficiency according to the concentration of NaCl during circular RNA synthesis using a human-derived RtcB RNA ligase.
FIG. 15 is a diagram confirming circular RNA synthesis efficiency according to a concentration of Mn during circular RNA synthesis using human-derived RtcB RNA ligase.
FIG. 16 is a graph showing circular RNA synthesis efficiency according to the concentration of DTT during circular RNA synthesis using human-derived RtcB RNA ligase.
FIG. 17 is a diagram confirming circular RNA synthesis efficiency according to a concentration of a 1:1 mixture of human-derived RtcB RNA ligase and Archease during circular RNA synthesis.
FIG. 18 is a diagram illustrating the circular RNA synthesis efficiency according to the concentration combinations of human-derived RtcB RNA ligase and Archease during circular RNA synthesis.
FIG. 19 is a diagram confirming circular RNA synthesis efficiency according to a ligation reaction temperature during circular RNA synthesis using RtcB RNA ligase derived from *Thermus thermophilus*.
FIG. 20 is a diagram confirming circular RNA synthesis efficiency according to a ligation reaction time during circular RNA synthesis using RtcB RNA ligase derived from *Thermus thermophilus*.
FIG. 21 is a diagram showing circular RNA synthesis efficiency using E. coli-derived RtcB RNA ligase according to the size of precursor RNA (nt).
FIG. 22 is a diagram showing the circular RNA synthesis efficiency using human-derived RtcB RNA ligase according to the size of precursor RNA (nt).
FIG. 23 is a diagram illustrating circular RNA synthesis efficiency using RtcB RNA ligase derived from *Thermus thermophilus* according to the size of precursor RNA (nt).
FIG. 24 is a diagram confirming the circular RNA synthesis efficiency using E. coli-derived RtcB RNA ligase according to the type of ribozyme included in the precursor RNA.
FIG. 25 is a diagram confirming circular RNA synthesis efficiency using human-derived RtcB RNA ligase according to a type of ribozyme included in precursor RNA.
FIG. 26 is a diagram showing the circular RNA synthesis efficiency using RtcB RNA ligase derived from *Thermus thermophilus* according to the type of ribozyme included in the precursor RNA.
FIG. 27 is a diagram confirming circular RNA synthesis efficiency according to the sequence of a homology region in the structure of the rpcRNA.
FIG. 28 is a diagram showing the structure of a precursor RNA template (rpc-sp-IH-sp template) that further comprises an internal homology region and a spacer in addition to the rpcRNA.
FIG. 29 is a diagram confirming the circular RNA synthesis efficiency of a precursor RNA, which further comprises an internal homology region and a spacer in rpcRNA, using E. coli-, human-, or *Thermus thermophilus*-derived RtcB RNA ligase.
FIG. 30 illustrates the *in vitro* confirmation of the expression of a target protein from a CDS included in rpcRNA and circular RNA.
FIG. 31 is a diagram confirming the expression of a target protein from a CDS included in rpcRNA and circular RNA *in vivo*.

### [BEST MODE FOR CARRYING OUT THE INVENTION]

Hereinafter, the present invention will be described in detail with embodiments of the present invention with reference to the accompanying drawings. However, the following embodiments are presented as examples of the present invention, and when it is determined that detailed descriptions of well-known techniques or configurations to those skilled in the art may unnecessarily obscure the gist of the present invention, the detailed descriptions thereof may be omitted, and the present invention is not limited thereby. Various modifications and applications of the present invention are possible within the description of the claims to be described later and the equivalent scope interpreted therefrom.

In addition, the terms (terminology) used in this specification are terms used to appropriately express preferred embodiments of the present invention, which may vary depending on the intention of a user or operator or customs in the field to which the present invention belongs. Therefore, definitions of these terms should be made based on the contents throughout this specification. Throughout the specification, when a certain part "includes" a certain component, this means that other components may be further included rather than excluding other components unless otherwise stated.

Unless otherwise indicated, a nucleic acid is written in a 5'→3' orientation from left to right. A numerical range listed within the specification includes numbers defining the range, and includes each integer or any non-integer fraction within the defined range.

All technical terms used in the present invention have the same meaning as generally understood by one of ordinary skill in the art to which the present invention pertains, unless otherwise defined. In addition, although preferred methods or samples are described in this specification, those similar or equivalent thereto also fall within the scope of the present invention. The contents of all publications cited as references in this specification are incorporated herein by reference.

In one aspect, the present invention relates to a circular RNA precursor having a structure of 5'-5' homology region-gene of interest (GOI)-3' homology region-ribozyme-3', wherein the 5' homology region includes a sequence that is a reverse complement to a 3' homology region.

In one embodiment, the 5' homology region may be a reverse complement to the 3' homology region.

In one embodiment, a length of a portion of the sequence of the 5' homology region that is reverse-complementary to the sequence of the 3' homology region may be 50% or more, 60% or more, 70% or more, or 80% or more of the total length of the 5' homology region.

In one embodiment, a reverse complement region of a sequence of each of the 5' homology region and the 3' homology region may be 50% or more, 60% or more, 70% or more, or 80% or more of the 5' sequence of the homology region.

In one embodiment, the 5' homology region may comprise a reverse complement sequence to a 3' sequence of a homology region, and may optionally comprise 1 to 5 mismatched nucleotide pairs in the reverse complement sequence.

In one embodiment, the 5' homology region may comprise a reverse complement sequence to a 3' sequence of a homology region, and the length of the reverse complement sequence may be 5 to 50 nt.

In one embodiment, the 5' homology region may further comprise GGX (wherein X is A, U, C, or G) at the 5' end, and the GGX may be GGC or GGG (Guanine).

In one embodiment, transcription efficiency from a template encoding the same to precursor RNA may be increased by the addition of the GGX at the 5' end.

In one embodiment, the 5' homology region may include a nucleotide sequence of SEQ ID NO: 1.

In one embodiment, the 5' homology region may be a reverse complement to the 3' homology region except for the GGX at the 5' end.

In one embodiment, the 5' homology region may comprise any one sequence selected from the group consisting of SEQ ID NOs: 1 to 4, and the 5' homology region may comprise any one sequence selected from the group consisting of SEQ ID NOs: 2 to 4 in a 3' direction from the GGX present at the 5' end of the 5' homology region.

In one embodiment, the 3' homology region may comprise any one sequence selected from the group consisting of SEQ ID NOs: 5 to 10.

In one embodiment, the circular RNA precursor may include a 5' homology region including a nucleotide sequence of SEQ ID NO: 2 and a 3' homology region including a nucleotide sequence of SEQ ID NO: 5; a 5' homology region including a nucleotide sequence of SEQ ID NO: 2 and a 3' homology region including a nucleotide sequence of SEQ ID NO: 6; a 5' homology region including a nucleotide sequence of SEQ ID NO: 2 and a 3' homology region including a nucleotide sequence of SEQ ID NO: 7; a 5' homology region including a nucleotide sequence of SEQ ID NO: 3 and a 3' homology region including a nucleotide sequence of SEQ ID NO: 8; or a 5' homology region including a nucleotide sequence of SEQ ID NO: 4 and a 3' homology region including a nucleotide sequence of SEQ ID NO: 9.

In one embodiment, a triphosphate (P-P-P) may exist at the 5' end of the circular RNA precursor.

In one embodiment, the gene of interest (GOI) may comprise a target gene or a gene encoding a protein, for example, a CDS (coding sequence), and the length of the gene of interest may be 100 to 9000 nt.

In one embodiment, the gene of interest may comprise an IRES (internal ribosome entry site) region at the 5' end, and the IRES may be an entire sequence, a partial sequence, or a variant of one or more virus-derived sequences selected from the group consisting of Taura syndrome virus, Triatoma virus, Theiler's encephalomyelitis virus, Simian Virus 40, Solenopsis invicta virus 1, Rhopalosiphum padi virus, Reticuloendotheliosis virus, Human poliovirus 1, Plautia stali intestine virus, Kashmir bee virus, Human rhinovirus 2, Homalodisca coagulata virus-1, Human Immunodeficiency Virus type 1, Homalodisca coagulata virus-1, Himetobi P virus, Hepatitis C virus, Hepatitis A virus, Hepatitis GB virus, Foot and mouth disease virus, Human enterovirus 71, Equine rhinitis virus, Ectropis obliqua picorna-like virus, Encephalomyocarditis virus, Drosophila C Virus, Human coxsackievirus B3, Cru- cifer tobamovirus, Cricket paralysis virus, Bovine viral diarrhea virus 1, Black Queen Cell Virus, Aphid lethal paralysis virus, Avian encephalomyelitis virus, Acute bee paralysis virus, Hibiscus chlorotic ringspot virus, Classical swine fever virus, Human FGF2, Human SFTPA1, Human AML1/RUNX1, Drosophila antennapedia, Human AQP4, Human ATIR, Human BAG-1, Human BCL2, Human BiP, Human c-IAP1, Human c-myc, Human eIF4G, Mouse NDST4L, Human LEF1, Mouse HIF1 alpha, Human n.myc, Mouse Gtx, Human p27kip1, Human PDGF2/c-sis, Human p53, Human Pim-1, Mouse Rbm3, Drosophila reaper, Canine Scamper, Drosophila Ubx, Human UNR, Mouse UtrA, Human VEGF-A, Human XIAP, Drosophila hairless, S. cerevisiae TFIID, S. cerevisiae YAP1, tobacco etch virus, turnip crinkle virus, EMCV-A, EMCV-B, EMCV-Bf, EMCV-Cf, EMCV pEC9, Picobirnavirus, HCV QC64, Human Cosavirus E/D, Human Cosavirus F, Human Cosavirus JMY, Rhinovirus NAT001, HRV14, HRV89, HRVC- 02, HRV-A21, Salivirus A SH1, Salivirus FHB, Salivirus NG-J1, Human Parechovirus 1, Crohivirus B, Ye-3, Rosa- virus M-7, Shanbavirus A, Pasivirus A, Pasivirus A 2, Echovirus E14, Human Parechovirus 5, Aichi Virus, Hepatitis A Virus HA16, Phopivirus, CVA10, Enterovirus C, Enterovirus D, Enterovirus J, Human Pegivirus 2, GBV-C GT110, GBV-C K1737, GBV-C Iowa, Pegivirus A 1220, Pasivirus A 3, Sapelovirus, Rosavirus B, Bakunsa Virus, Tremovirus A, Swine Pasivirus 1, PLV-CHN, Pasivirus A, Sicinivirus, Hepacivirus K, Hepacivirus A, BVDV1, Border Disease Virus, BVDV2, CSFV-PK15C, SF573 Dicistrovirus, Hubei Picoma-like Virus, CRPV, Salivirus A BN5, Salivirus A BN2, Salivirus A 02394-01, Salivirus A GUT, Salivirus A CH, Salivirus A SZ1, Salivirus FHB, Salivirus NG-J1, Salivirus A YZ01, Salivirus A YZ02, Salivirus A YZ03, Salivirus A FUAN-01, Salivirus sp isolate ETH, Salivirus A Hu/Baja-5332, Salivirus sp isolate CHN/XJ/env, CVB3, CVB1, Echovirus 7, CVBS, EVA71, CVA3, CVA12, and EV24.

In one embodiment, the IRES may be an aptamer for elF4G (aptamer).

In one embodiment, the circular RNA precursor may further comprise a 5' internal homology region in a 3' direction of the 5' homology region, and a 3' internal homology region in a 5' direction of the 3' homology region, wherein the 5' internal homology region may comprise a sequence that is a reverse complement to the 3' internal homology region, which has the effect of increasing the synthesis efficiency of circular RNA having a long length.

In one embodiment, the 5' internal homology region may include a nucleotide sequence of SEQ ID NO: 10.

In one embodiment, a spacer region may be further included in a 5' direction and a 3' direction of the internal homology region, and the spacer may include a nucleotide sequence of SEQ ID NO: 11 or 12.

In one embodiment, the ribozyme may be a small ribozyme of 30 to 150 nt, and may be a Twister, Twister Sister, Hatchet, Pistol, Hammerhead, Hairpin, Hepatitis Delta Virus (HDV), Neurospora Varkud Satellite (VS), Vg1, glucosamine-6-phosphate synthase (glmS), or an engineered synthetic ribozyme, or a variant thereof, and may include a nucleotide sequence of SEQ ID NO: 13.

In one embodiment, the circular RNA precursor may further comprise a label at the 3' end, and the label may be a PolyA, PolyG, PolyU, or PolyC sequence.

In one embodiment, the circular RNA precursor may have a size of about 250 to 10,000 nt.

In one embodiment, the circular RNA precursor may include a nucleotide analog in which a sugar or backbone of one or more nucleotides is modified.

In one embodiment, the sugar modification may be the substitution of the hydroxyl group (-OH) at the 2' carbon position of ribose with a methyl group (-CH₃)(OMe), a methoxy group (-OCH₃), an amine group (-NH₂), fluorine (-F)(fluoro), an O-2-methoxyethyl group (methoxyethyl, MOE), an O-propyl group, an O-2-methylthioethyl group, an O-3-aminopropyl group, an O-3-dimethylaminopropyl group, an O-N-methylacetamido group, or an O-dimethylamidooxyethyl group, and it is more preferable that the 2'-hydroxyl group of ribose is substituted with 2'-MOE (2'-O-methoxyethyl), 2'-OMe (2'-O-Methyl), or 2'-F (fluoro).

In one embodiment, the modification of the backbone may be that the phosphate backbone of a nucleotide is modified with a phosphorothioate, a phosphorodithioate, a methyl phosphonate, an alkylphosphonate, a phosphoroamidate, or a boranophosphate, with modification with phosphorothioate being more preferred.

The term "reverse complement sequence" used in the present invention refers to the sequence obtained by taking the complementary sequence of a nucleotide sequence, which is represented from 5' to 3', and expressing that complementary sequence in the 5' to 3' direction. In general, the nucleotide sequence of a gene is represented in the 5' to 3' direction starting from the promoter position. During transcription, the complementary sequence of the nucleotide sequence represented in the 5' to 3' direction (i.e., the 3' to 5' sequence) is used as a template, and as a result, the 5' to 3' sequence from the promoter position matches the sequence of the mRNA transcript. Herein, the sequence identical to the mRNA is referred to as a sense sequence, and the sequence complementary to the sense sequence is referred to as an antisense sequence. The sequence of the strand used as a template, when expressed in the 5' to 3' direction, is referred to as a reverse complement sequence or a reverse complementation sequence. That is, when a nucleotide sequence is represented as 5'-AAC ACG GAC-3', its reverse complement sequence is 5'-GUC CGU GUU-3'.

The term "coding sequence (CDS)" as used in the present invention refers to a sequence that is translated into a protein.

The term "circular RNA precursor" used in the present invention is a linear single-stranded ribonucleotide before being circularized.

In one aspect, the present invention relates to a recombinant nucleic acid vector for generating the circular RNA precursor of the present invention.

In one embodiment, the recombinant nucleic acid vector may further comprise an RNA polymerase promoter sequence operably linked to a coding sequence of the circular RNA precursor.

In one embodiment, the recombinant nucleic acid vector may be a recombinant vector that transcribes the circular RNA precursor.

In one embodiment, the recombinant vector may include an operably linked promoter and a gene encoding the circular RNA precursor.

In one embodiment, the recombinant vector may include a template DNA of the circular RNA precursor.

The recombinant nucleic acid vector of the present invention may be produced by recombinant DNA methods known in the art, and for efficient transcription of the circular RNA precursor, it may further include regulatory sequences including a leader sequence, a polyadenylation sequence, a promoter, an enhancer, an upstream activation sequence, a signal peptide sequence, and a transcription terminator as needed.

In one aspect, the present invention relates to a composition for producing circular RNA, comprising the circular RNA precursor of the present invention, a phosphatase, and an RtcB RNA ligase.

In one aspect, the present invention relates to a kit for producing circular RNA, comprising the circular RNA precursor of the present invention, a phosphatase, and an RtcB RNA ligase.

In one aspect, the present invention relates to a method for producing circular RNA, comprising: a step of synthesizing a precursor RNA by in vitro transcription (IVT) of the vector of the present invention; a step of treating the synthesized precursor RNA with a phosphatase; and a step of treating the synthesized precursor RNA with an RtcB RNA ligase.

In one embodiment, the phosphatase and RtcB RNA ligase may be treated simultaneously or sequentially.

In one embodiment, a triphosphate at the 5' end of the circular RNA precursor may be made into a hydroxyl group (-OH) by the phosphatase treatment.

In one embodiment, a cyclic phosphate (2', 3' cyclic phosphate) may be induced at the 3' end by a ribozyme of the transcribed precursor RNA.

In one embodiment, the RtcB RNA ligase may circularize the circular RNA precursor by connecting the hydroxyl group of the 5' end of the circular RNA precursor and the cyclic phosphate at the 3' end.

In one embodiment, the method of the present invention may produce a circular RNA having a size of about 200 to 9950 nt.

In one embodiment, the RtcB RNA ligase may be derived from bacteria or mammals, and may be an E. coli-, *Thermus thermophilus*-, or human-derived RtcB RNA ligase.

In one embodiment, the method of the present invention may further comprise treating with Archease in the step of treating with an RtcB RNA ligase, and in this case, the method has the effect of improving the circularization efficiency of a larger/longer circular RNA precursor.

In one embodiment, the method of the present invention may further comprise the step of treating with an exoribonuclease after the RtcB RNA ligase treatment step.

In one aspect, the present invention relates to the use of a circular RNA precursor for producing circular RNA.

### [MODE FOR CARRYING OUT THE INVENTION]

The present invention will be described in more detail through the following examples. However, the following examples are only for clarifying the contents of the present invention, and the present invention is not limited thereby.

### Example 1. Synthesis of precursor RNA

### 1-1. Precursor RNA structure design

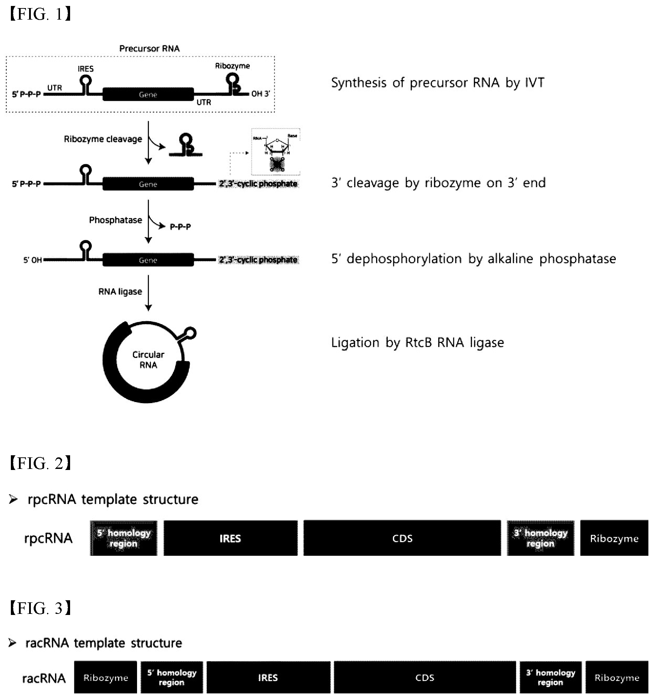

In order to use an RtcB RNA ligase that recognizes a -OH at the 5' end and a cyclic phosphate (2', 3' cyclic phosphate) at the 3' end, a precursor rpcRNA template structure comprising a 5' homology region-IRES-CDS (target sequence)-3' homology region-3' ribozyme from the 5' end was designed (FIG. 2). In addition, for comparison with the rpcRNA, a conventional precursor racRNA template structure comprising a ribozyme at the 5' end was designed (FIG. 3).

### 1-2. Precursor RNA template synthesis

After the IVT template DNA for synthesizing precursor RNA by in vitro transcription (IVT) was synthesized with the structure designed in Example 1-1, the synthesized DNA was transformed into DH5α chemically competent E. coli cells (Enzynomics, CP010), spread on a 50 µg/ml kanamycin LB plate, and cultured overnight in an incubator at 37 °C. One colony generated on the plate was inoculated into 50 µg/ml kanamycin LB broth and cultured with shaking at 200 rpm at 37 °C. Thereafter, cells were harvested and disrupted, the IVT template DNA was purified using a QIAfilter plasmid Midi kit (Qiagen, 12243), and the purified IVT template was linearized using restriction enzyme XbaI under the conditions of Table 1. The linearized IVT template DNA was purified with a Qiaquick PCR purification kit (Qiagen, 28106).

**[Table 1]**

| **Component** | **Condition** |
|---|---|
| IVT template DNA | 15 ug |
| 10 x buffer | 10 ul |
| Restrction enzyme (20 U/ul) | 4 ul |
| D.W | Up to 100 ul |

### 1-3. Precursor RNA synthesis

Precursor RNA was synthesized using the linearized IVT template DNA synthesized in Example 1-2 and an EZ^{™} MEGA T7 transcription kit (Enzynomics, EZ039S). Specifically, 1 µg of IVT template DNA, 2 µl of MEGA T7 enzyme Mix, 2 µl of 10x MEGA T7 reaction buffer, 2 µl of 100 mM rATP, 2 µl of 100 mM rCTP, 2 µl of 100 mM rGTP, and 2 µl of 100 mM rUTP were added to a PCR strip-tube (Axygen, PCR-0208-CP-C) to reach a final volume of 20 µl and mixed. After reacting the mixture at 37°C for 2 hours using a SimpliAmp Thermal Cycler (Thermo), 2 µl of DNase I was added, and the mixture was reacted at 37°C for 15 minutes. After adding 80 µl of NFW to the reaction mixture and mixing, the synthesized precursor RNA was purified using an RNeasy Mini Kit (Qiagen, 74106), thereby obtaining two types of racRNA precursors (racRNA and racRNA2) and two types of rpcRNA precursors (rpc-1 and rpc-3'rib), each comprising the sequences of Table 2 below.

**[Table 2]**

| | **5' ribozyme** | **5' homology region** | **GOI** | **3' homology region** | **3' ribozyme** |
|---|---|---|---|---|---|
| **racRNA** | GCCAU CAGUC GCCGG UCCCA AGCCC GGAUA AAAUG GGAGG GGGCG GGAAA CCGCC U | AACC AUGCC GACUG AUGGC AG | IRES CDS | CUGC CAUCA GUCGG CGUGG ACUGU AG | ACUGU AG AAC ACUGC CAAUG CCGGU CCCAA GCCCG GAUAA AAGUG GAGGG UACAG UCCAC GC |
| **racRNA2** | GCCAU CAGUC GCCGG UCCCA AGCCC GGAUA AAAUG GGAGG GGGCG GGAAA CCGCC U | AACC AUGCC GACUG AUGGC AG | IRES CDS | CUGC CAUCA GUCGG CGUGG ACAGU AG | ACUGU AG AAC ACUGC CAAUG CCGGU CCCAA GCCCG GAUAA AAGUG GAGGG UACUG UCCAC GC |
| **rpc-1** | | GGG AAC ACG GAC UCA AGC UCC UAC | IRES CDS | GUA GGA GCU UGA GUC CGU GUU | GAC AGU AG AAC ACUGC CAAUG CCGGU CCCAA GCCCG GAUAA AAGUG GAGGG UACUG UC |
| **rpc-3'rib** | | GGCC AUGCC GACUG AUGGC AG | IRES CDS | CUGC CAUCA GUCGG CGUGG ACAGU AG | ACAGU AG AAC ACUGC CAAUG CCGGU CCCAA GCCCG GAUAA AAGUG GAGGG UACUG UCCAC GC |

### 1-4. Analysis of transcription efficiency of precursor RNA according to sequence of a homology region

In order to confirm the transcription efficiency according to the 5' terminal start sequence of the 5' homology region of a precursor rpcRNA template structure in the precursor RNA synthesis of Example 1-3, that is, in the transcription step, the yield per IVT 1 reaction of templates encoding the following was analyzed: two types of racRNA, which are conventional precursor RNAs that must have a GXX sequence due to a ribozyme sequence located at the 5' (racRNA and racRNA2 having a GCC sequence as the 5' terminal start sequence of the 5' homology region and different subsequent sequences of the homology region), and two types of rpcRNA of the present invention having a GGX sequence (rpc-3'rib having a GGC sequence as the 5' terminal start sequence of the 5' homology region and rpc-1 having a GGG sequence).

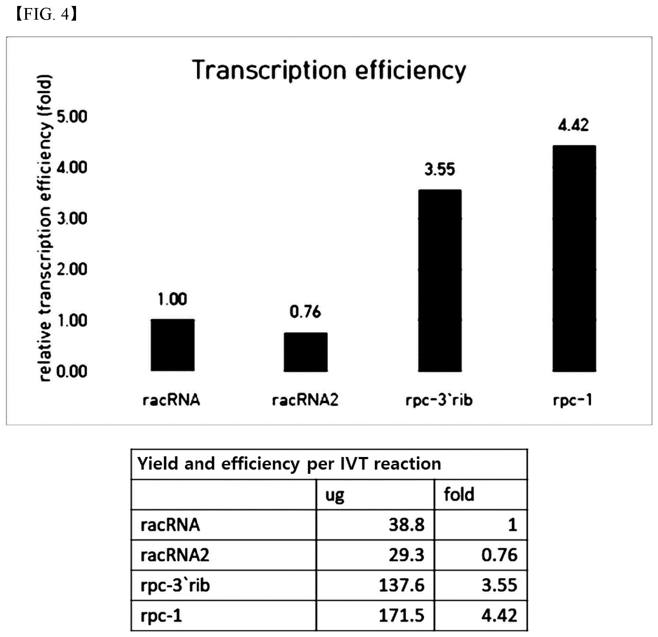

As a result, the yields per IVT 1 reaction of racRNA, racRNA2, rpc-3'rib, and rpc-1 were 38.8 µg, 29.3 µg, 137.6 µg, and 171.5 µg, respectively (FIG. 4), and it was found that the transcription efficiency of rpcRNA comprising the GGX sequence was about 3.55 to 4.42 times higher than that of racRNA comprising the GXX sequence. Therefore, it was confirmed that the efficiency of the *in vitro* transcription (IVT) step was increased by the sequence configuration described above, whereby the overall circular RNA production efficiency may be improved.

### Example 2. Dephosphorylation of the 5'-end of precursor RNA

Since RtcB RNA ligase acts by recognizing a hydroxyl group at the 5' end and a cyclic phosphate at the 3' end, a triphosphate (P-P-P) group existing at the 5' end of a precursor RNA was dephosphorylated using a phosphatase to convert the 5' end into a hydroxyl group (-OH). Specifically, among the precursor RNAs synthesized in Example 1, 10 µL of each of rpc-1 and rpc-3`rib, which do not have a ribozyme at the 5' end, were treated by adding 10x Antarctic Phosphatase buffer and 50 units of Antarctic Phosphatase (NEB, M026L) to reach a final volume of 100 µL, and then the mixture was incubated at 37°C for 30 minutes to perform dephosphorylation. The dephosphorylated precursor RNA was purified using an RNeasy Mini Kit (Qiagen, 74106).

### Example 3. Circular RNA synthesis using RtcB RNA ligase (ligase)

### 3-1. Circular RNA synthesis

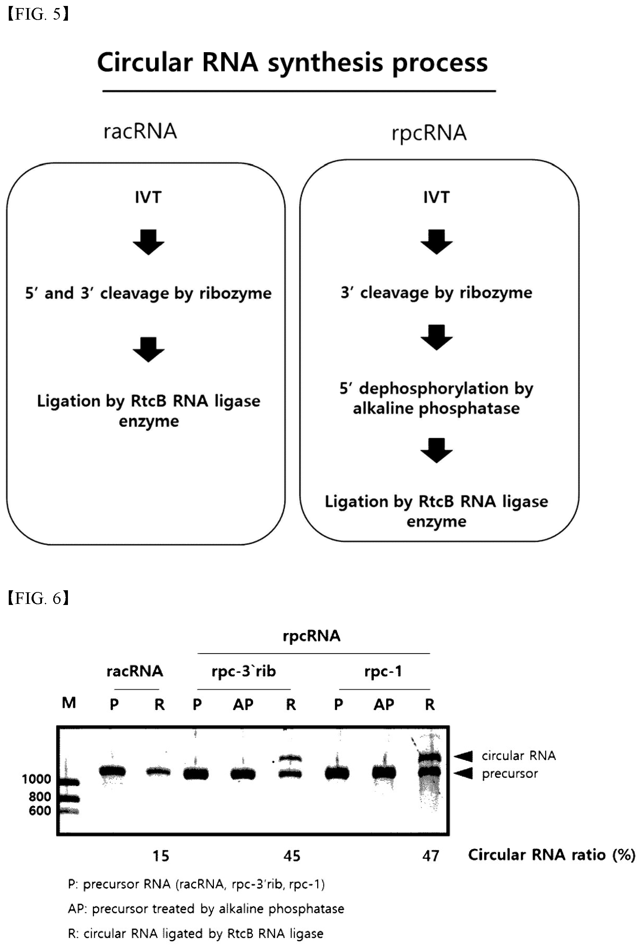

The racRNA synthesized in Example 1 and the dephosphorylated precursor RNA prepared using a phosphatase in Example 2 were each circularized using RtcB RNA ligase (Enzynomics Cat#. M064L) (FIG. 5). Specifically, 5 pmol of precursor RNA racRNA or dephosphorylated precursor RNA (rpc-1 or rpc-3`rib), 2 µl of 10x RtcB reaction buffer, 20 nM of GTP, 0.2 mM of MnCl₂, and 15 pmol of RtcB ligase were added to a PCR strip-tube and mixed to a final volume of 20 µl. After reacting at 37°C for 15 minutes using a SimpliAmp Thermal Cycler (Thermo), the synthesized circular RNA was purified using RNA Clean & Concentrator-5 (Zymo Research, R1015).

### 3-2. Analysis of circular RNA

The circular RNA (rpc-1, rpc-3`rib, and racRNA) (R) synthesized in Example 3-1, the precursor RNA (rpc-1, rpc-3`rib, and racRNA) (P) synthesized in Example 1, and the dephosphorylated precursor RNA (rpc-1 and rpc-3`rib) (AP) synthesized in Example 2 were analyzed by electrophoresis using an E-Gel^{™} Power Snap Electrophoresis System (Thermo). Specifically, 200 ng of each RNA, 10 µl of D.W, and 10 µl of formamide (Sigma, F9037) were added to a PCR strip-tube and mixed. The mixture was reacted at 70°C for 5 minutes using a SimpliAmp Thermal Cycler (Thermo), cooled on ice for 3 minutes, and the sample and a ladder (a mixture of 1 µl of RiboRuler High Range RNA ladder (Thermo, SM1821), 9 µl of D.W, and 10 µl of formamide) were loaded into a 2% E-gel EX (Thermo, G401002) mounted on an E-Gel^{™} Power Snap Electrophoresis Device (Thermo, G8100) and run using the E-Gel EX 1-2% method. After the run, the gel was cooled for 15 minutes, and the bands were imaged using a ChemiDoc^{™} XRS+ System (Bio-rad, 1708265). As a result, it was found that the circular RNA ratio of rpc-1 (47%) and rpc-3`rib (45%) was significantly higher than that of the precursor RNA racRNA (15%) (FIG. 6), and it was confirmed that converting the 5' end of the precursor RNA into a hydroxyl group using a phosphatase is important for the circularization of the precursor RNA.

### Example 4. Circular RNA synthesis efficiency analysis according to ligation reaction conditions

### 4-1. Circular RNA synthesis efficiency analysis using E. coli-derived RtcB RNA ligase

### 4-1-1. Analysis of circularization efficiency according to synthesis reaction conditions

In Example 2, dephosphorylated precursor RNA rpcRNA(rpc-1) (IRES: Salivirus FHB and CDS: Gaussia luciferase), which was prepared using a phosphatase, was circularized with E. coli-derived RtcB RNA ligase. The circularization efficiency (circular RNA synthesis efficiency) was confirmed according to reaction conditions including the concentration of GTP (10 to 100 nM), the concentration of Mn (0.05 to 0.5 nM), the combination of Mn and GTP concentrations (GTP: 20 to 100 nM, MnCl₂: 0.1 to 1 mM), and the incubation temperature (25 to 37°C) and time (0 to 60 minutes). Specifically, 5 pmol of rpcRNA, 2 µl of 10x RtcB reaction buffer, 15 pmol of E. coli RtcB RNA ligase (Cat#. M064L, Enzynomics), 10 to 100 nM of GTP, and 0.05 to 0.5 nM of MnCl₂ were mixed in a PCR strip-tube to a total volume of 20 µl. The mixture was reacted by incubating at a temperature of 25 to 37°C for 0 to 60 minutes using a SimpliAmp Thermal Cycler (Thermo). Thereafter, the synthesized circular RNA was purified using RNA Clean & Concentrator-5 (Zymo Research, R1015), and 200 ng of the purified circular RNA was confirmed by electrophoresis.

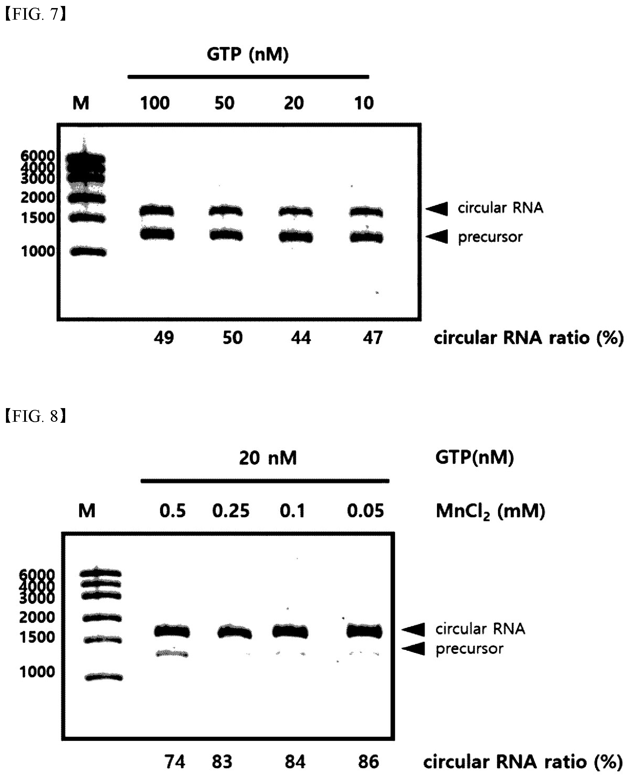

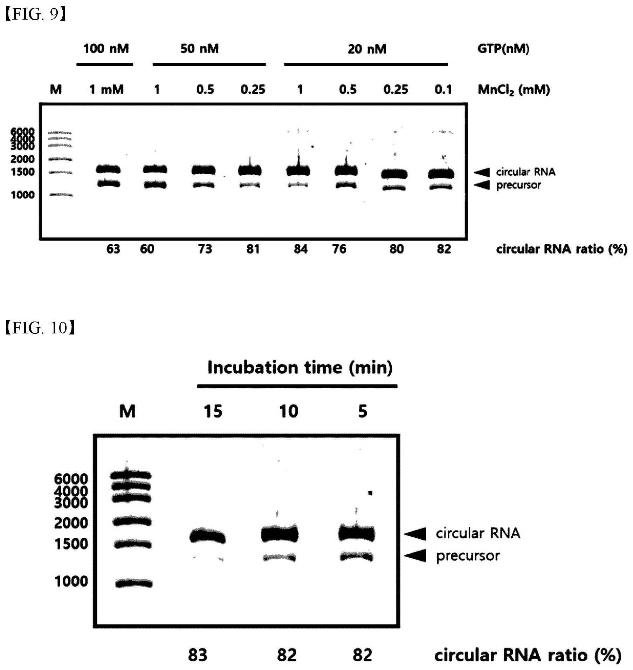

As a result, it was found that the circular RNA synthesis efficiency was highest under conditions of reacting at 37°C for 15 minutes with a composition comprising 20 nM GTP and 0.2 mM MnCl₂ (FIGS. 7 to 11).

### 4-1-2. Confirmation of circular RNA synthesis

In order to confirm whether the synthesized circular RNA is actually a circular RNA that is not degraded by an exoribonuclease, the circular RNA synthesized in Example 4-1-1 was treated with RNase R for 0 to 60 minutes, and the results were confirmed by electrophoresis.

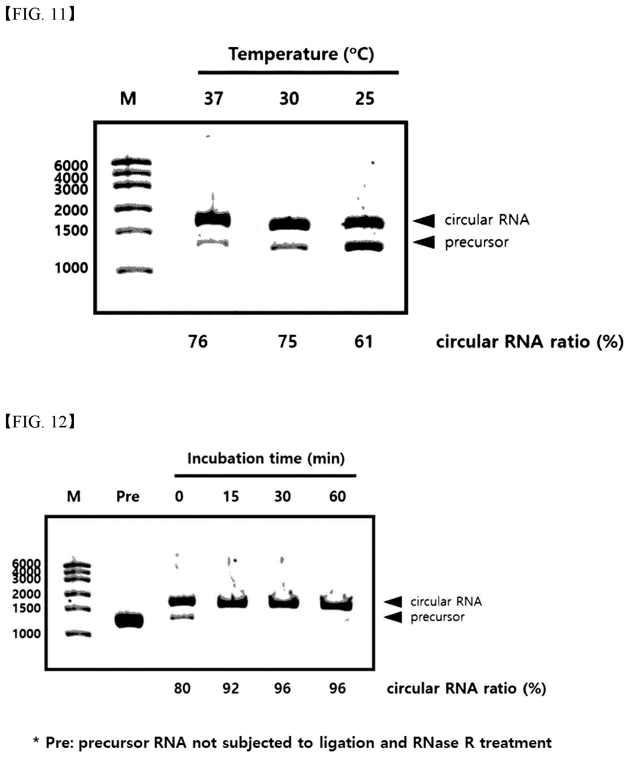

As a result, it was found that the newly synthesized RNA was not degraded even after RNaseR treatment (FIG. 12), and it was thereby confirmed that the synthesized RNA was a circular RNA.

### 4-2. Circular RNA synthesis efficiency analysis using human-derived RtcB RNA ligase

### 4-2-1. Analysis of circularization efficiency according to synthesis reaction conditions

In Example 2, circularization efficiency (circular RNA synthesis efficiency) was evaluated by circularizing dephosphorylated precursor RNA rpcRNA(rpc-1) (IRES: Salivirus FHB and CDS: IgG HC) using human-derived RtcB RNA ligase under various conditions including a concentration of GTP (1.0 to 1 mM), a concentration of NaCl (70 to 300 mM), a concentration of MnCl₂ (0.2 to 1 mM), and a concentration of DTT (0 to 10 mM). Specifically, 12.5 pmol of rpcRNA, 5 µl of 10x RtcB reaction buffer, 500 nM human RtcB ligase (Sinobio), 1.0 to 1 mM of GTP, 70 to 300 mM of NaCl, 0.2 to 1 mM of MnCl₂, and 0 to 10 mM of DTT were mixed in a PCR strip-tube (Axygen, PCR-0208-CP-C) to a total volume of 50 µl. The mixture was reacted by incubation at 37°C for 30 minutes using a SimpliAmp Thermal Cycler (Thermo). Thereafter, the synthesized circular RNA was purified using RNA clean & concentrator-5 (Zymo research, R1015), and 200 ng of the purified circular RNA was confirmed by electrophoresis.

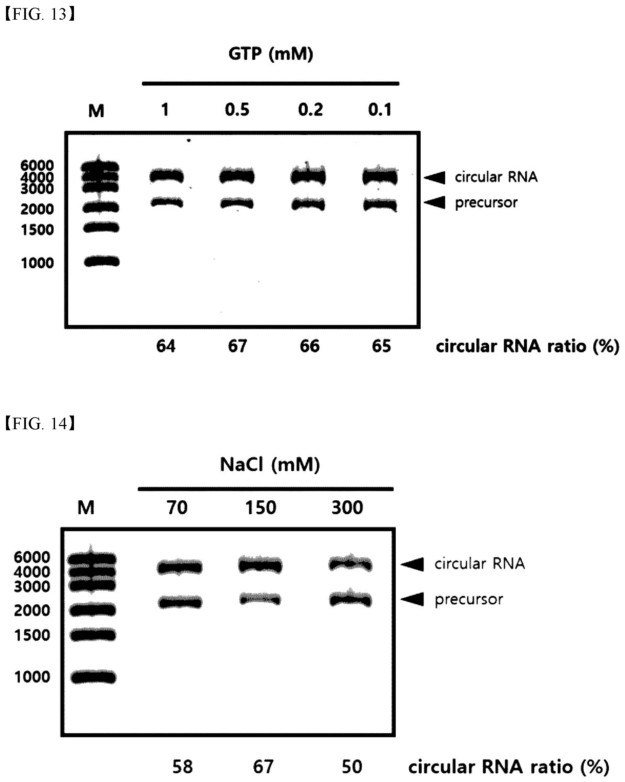

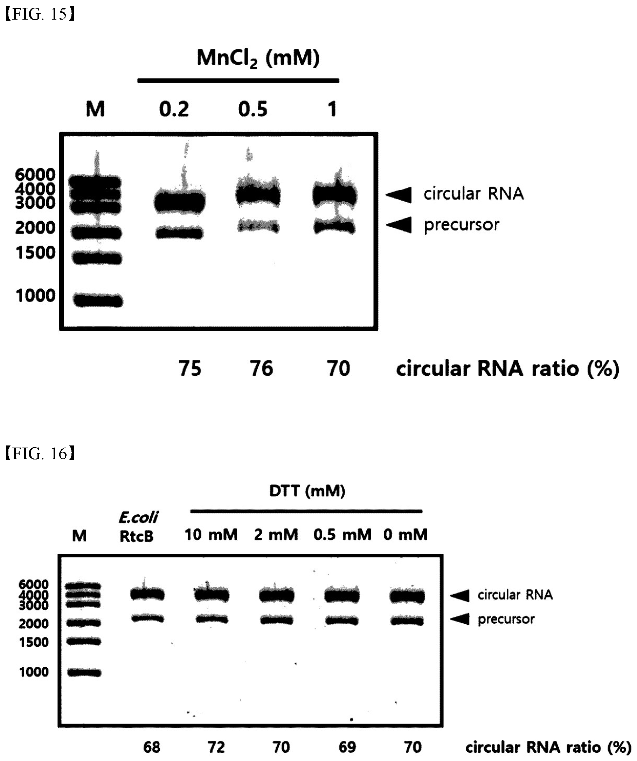

As a result, the circular RNA synthesis efficiency was found to be the highest under the condition of a composition containing 0.5 mM GTP and 0.5 mM MnCl₂ (FIGS. 13 to 16).

### 4-2-2. Combination of human-derived RtcB RNA ligase and Archease

Archease was used in combination as an enhancer of RNA ligase, and the circular RNA synthesis efficiency was analyzed. Specifically, human RtcB RNA ligase and Archease were mixed in a 1:1 molar ratio and treated at a concentration of 250 nM to 4 µM, or 1 to 5 µM of Archease was combined with 250 or 500 nM of human RtcB RNA ligase for treatment.

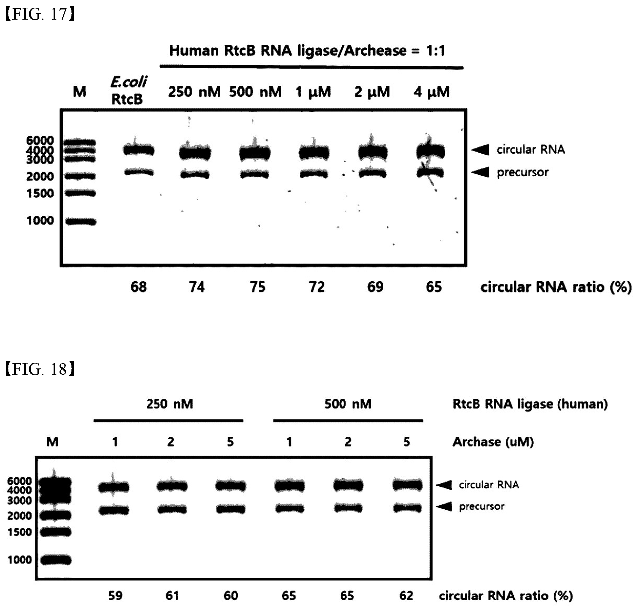

As a result, it was found that the circular RNA synthesis efficiency was the highest under the combination condition of 500 nM human RtcB ligase and 500 nM human Archease (FIGS. 17 and 18).

### 4-3. Circular RNA synthesis efficiency analysis using RtcB RNA ligase derived from Thermus thermophilus

In Example 2, the circularization efficiency (circular RNA synthesis efficiency) according to incubation temperature and time conditions was confirmed while circularizing a dephosphorylated precursor RNA, rpcRNA(rpc-1) (IRES: Salivirus FHB and CDS: Gaussia luciferase), using RtcB RNA ligase derived from *Thermus thermophilus*. Specifically, 10 pmol of rpcRNA, 2 µl of 5x RtcB reaction buffer, 0.15 mM of GTP, 0.25 mM of MnCl₂, 2.5 µM of T. thermophilus RtcB ligase, and T. thermophilus Archease were mixed in a PCR strip-tube (Axygen, PCR-0208-CP-C) to a total volume of 20 µl. The mixture was reacted by incubating for 10 to 60 minutes at a reaction temperature of 50 to 60°C using a SimpliAmp Thermal Cycler (Thermo). Thereafter, the synthesized circular RNA was purified using RNA clean & concentrator-5 (Zymo research, R1015), and 200 ng of the purified circular RNA was confirmed by electrophoresis.

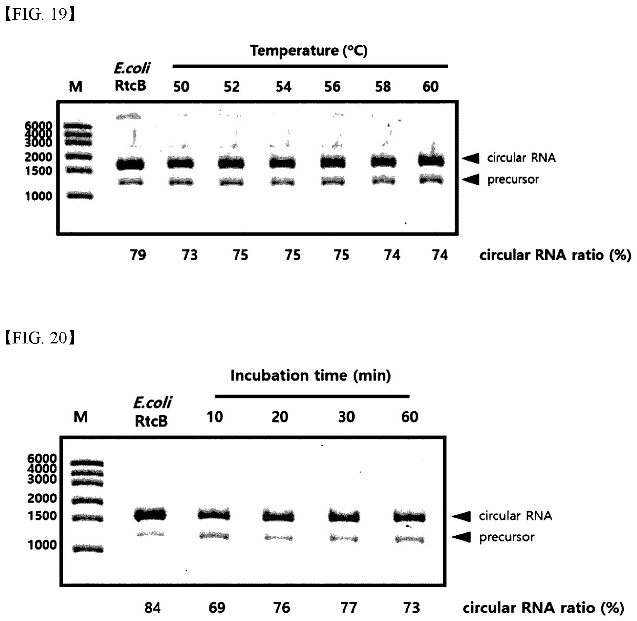

As a result, it was found that the circular RNA synthesis efficiency was highest under the condition of reacting at 55°C for 20 minutes (FIGS. 19 to 20).

### Example 5. Circular RNA synthesis efficiency analysis according to circular RNA size

### 5-1. Circular RNA synthesis efficiency analysis using E. coli RtcB RNA ligase

In order to analyze circular RNA synthesis efficiency according to the size of the precursor RNA, rpcRNA(rpc-1) of 1390, 1489, 2176, or 3016 nt, which had been dephosphorylated using a phosphatase by the method of the present invention, was subjected to circular RNA synthesis using E. coli-derived RtcB RNA ligase under the conditions optimized in Example 4-1-1, and the synthesized circular RNA was compared with circular RNA synthesized using racRNA of 1390 nt.

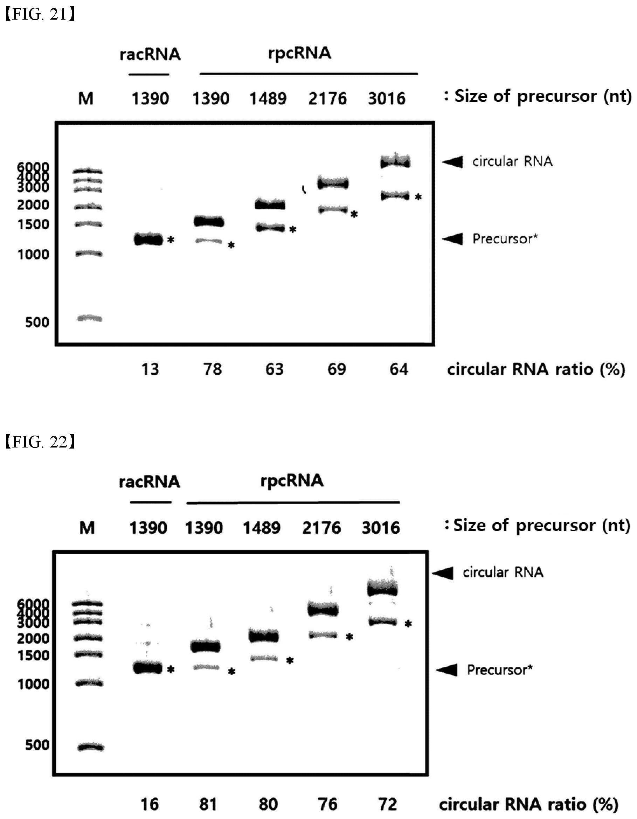

As a result, when synthesized by the method of the present invention using rpcRNA, it was observed that the circular RNA synthesis efficiency was significantly increased compared to the conventional racRNA, and it was further observed that the circular RNA synthesis efficiency was significantly increased even when the size of the precursor RNA was increased (FIG. 21).

### 5-2. Circular RNA synthesis efficiency analysis using human RtcB RNA ligase

In order to analyze circular RNA synthesis efficiency according to the size of the precursor RNA, circular RNAs were synthesized using human-derived RtcB RNA ligase for dephosphorylated rpcRNA (rpc-1) of 1390, 1489, 2176, or 3016 nt, which had been dephosphorylated using a phosphatase by the method of the present invention under the conditions optimized in Example 4-2, and compared with circular RNA synthesized using 1390 nt racRNA.

As a result, when circular RNA was synthesized by the method of the present invention using rpcRNA, it was found that the circular RNA synthesis efficiency significantly increased compared to the conventional racRNA, and it was found that the circular RNA synthesis efficiency significantly increased even when the size of the precursor RNA was increased (FIG. 22).

### 5-3. Circular RNA synthesis efficiency analysis using Thermus thermophilus RtcB RNA ligase

In order to analyze the circular RNA synthesis efficiency according to the size of the precursor RNA, circular RNAs were synthesized from 1390, 1489, 2176, or 3016 nt rpcRNA (rpc-1), which had been dephosphorylated using a phosphatase according to the method of the present invention, using RtcB RNA ligase derived from *Thermus thermophilus* under the conditions optimized in Example 4-3, and compared with the circular RNA synthesized using 1390 nt racRNA.

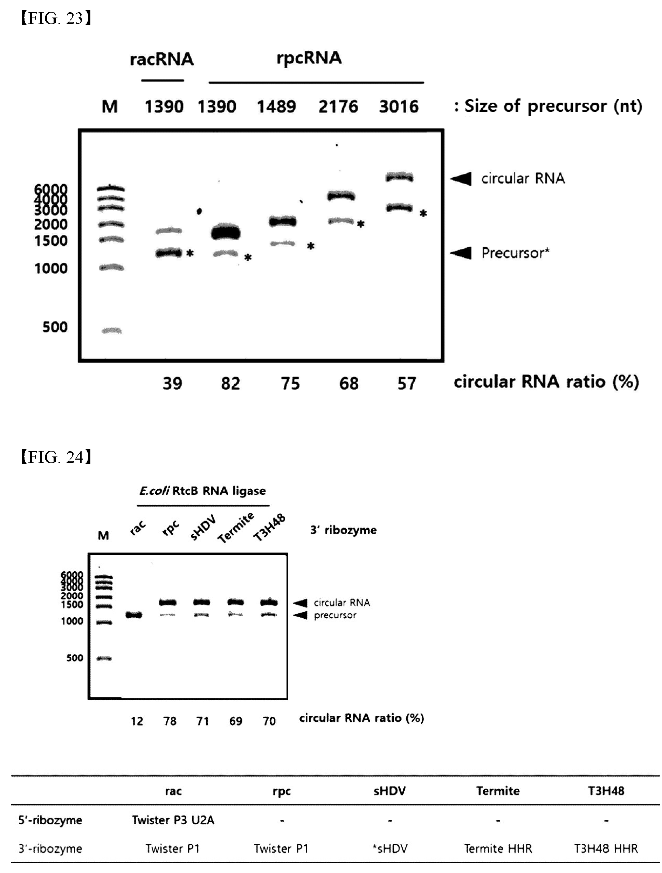

As a result, when synthesized by the method of the present invention using rpcRNA, the circular RNA synthesis efficiency was found to be significantly increased compared to that of conventional racRNA, and the circular RNA synthesis efficiency was found to be significantly increased even when the size of the precursor RNA was increased (FIG. 23).

### Example 6. Circular RNA synthesis efficiency analysis according to 3' terminal ribozyme type

### 6-1. Circular RNA synthesis efficiency analysis using E. coli RtcB RNA ligase

In order to analyze the circular RNA synthesis efficiency according to the type of ribozyme located at the 3' end of the precursor RNA, rpcRNA (IRES: FHB and CDS: Gaussia luciferase) comprising Twister P1, sHDV (short HDV (hepatitis delta virus-derived ribozyme) ribozyme), Termite HHR (hammerhead ribozyme), or T3H48 HHR at the 3' end was dephosphorylated using a phosphatase, and circular RNA was synthesized using E. coli-derived RtcB RNA ligase under the conditions optimized in Example 4-1-1. At this time, for comparison, the resulting circular RNA was compared with circular RNA synthesized using racRNA (IRES: Salivirus FHB and CDS: Gaussia luciferase) comprising Twister P3 U2A at the 5' end and Twister P1 ribozyme at the 3' end.

As a result, when synthesized by the method of the present invention using rpcRNA (rpc-1), it was found that the circular RNA synthesis efficiency was significantly increased in all ribozymes compared to the conventional racRNA (FIG. 24).

### 6-2. Circular RNA synthesis efficiency analysis using human RtcB RNA ligase

In order to analyze circular RNA synthesis efficiency according to the type of ribozyme located at the 3' end of the precursor RNA, rpcRNA (rpc-1) (IRES: Salivirus FHB and CDS: Gaussia luciferase) comprising Twister P1, sHDV, Termite HHR, or T3H48 HHR at the 3' end was dephosphorylated using a phosphatase, and circular RNA was synthesized using human-derived RtcB RNA ligase under the conditions optimized in Example 4-2. For comparison, the synthesized circular RNA was compared with circular RNA synthesized using racRNA (IRES: FHB and CDS: Gaussia luciferase) comprising Twister P3 U2A at the 5' end and Twister P1 ribozyme at the 3' end.

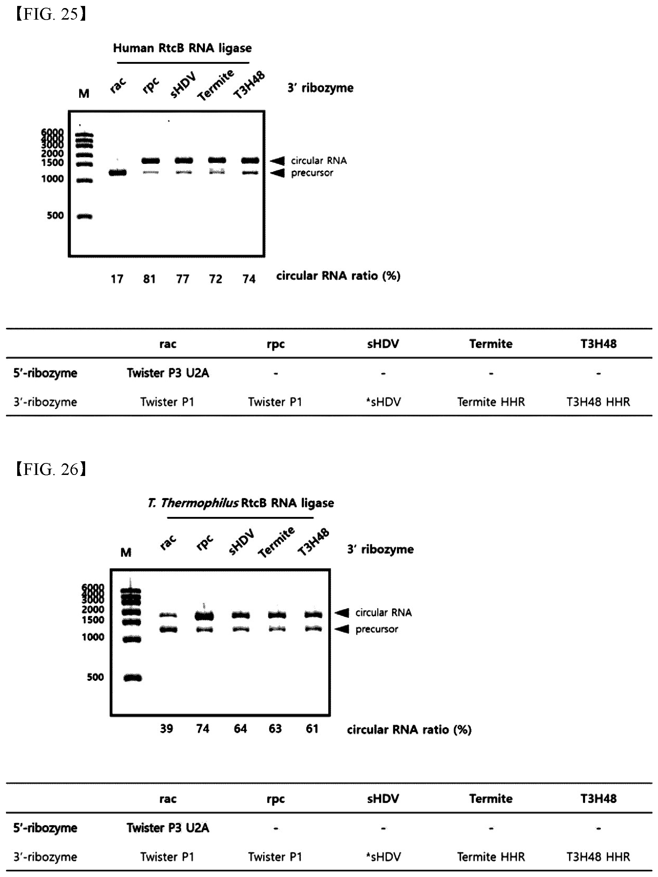

As a result, when synthesized by the method of the present invention using rpcRNA, it was found that the circular RNA synthesis efficiency was significantly increased in all ribozymes compared to that of conventional racRNA (FIG. 25).

### 6-3. Circular RNA synthesis efficiency analysis using Thermus thermophilus RtcB RNA ligase

In order to analyze the circular RNA synthesis efficiency according to the type of ribozyme located at the 3' end of the precursor RNA, rpcRNA(rpc-1) (IRES: Salivirus FHB and CDS: Gaussia luciferase), which includes Twister P1, sHDV, Termite HHR, or T3H48 HHR at the 3' end, was dephosphorylated using a phosphatase, and circular RNA was synthesized using human-derived RtcB RNA ligase under the conditions optimized in Example 4-2. In this case, for comparison, the synthesized circular RNA was compared with circular RNA synthesized using racRNA (IRES: FHB and CDS: Gaussia luciferase) including Twister P3 U2A at the 5' end and Twister P1 ribozyme at the 3' end.

As a result, when synthesized by the method of the present invention using rpcRNA, the circular RNA synthesis efficiency was found to be significantly increased for all ribozymes compared to the conventional racRNA (FIG. 26).

### Example 7. Circular RNA synthesis efficiency analysis according to homology region sequence

In order to analyze the circular RNA synthesis efficiency according to the sequence of a homology region in the structure of the rpcRNA of the present invention, the 5' homology region of the rpcRNA (rpc-1), excluding the GGX (GGC or GGG) at the 5' end, was configured with the sequences in Table 3 below. Then, the circular RNA synthesis efficiency was compared after either changing the 3' sequence of a homology region to have 1 to 2 nucleotide mismatches with the 5' homology region (M1 and M2), or configuring the lengths of the 5' homology region and the 3' homology region to be 3 to 5 nucleotides shorter, respectively (D6 and D10).

**[Table 3]**

| **Homology sequence** | | |
|---|---|---|
| | **5' Homology region** | **3' Homology region** |
| **rpc** | AAC ACG GAC UCA AGC UCC UAC | GUA GGA GCU UGA GUC CGU GUU |
| **M1** | AAC ACG GAC UCA AGC UCC UAC | GUA GGA GAU UGA GUC CGU GUU |
| **M2** | AAC ACG GAC UCA AGC UCC UAC | GUA GGA GAU UGA GUA CGU GUU |
| **D6** | AAC ACG GAC UCA AGC UAC | GUA GCU UGA GUC CGU GUU |
| **D10** | AAC ACG GAC UCA AUA C | GUA UUG AGU CCG UGU U |

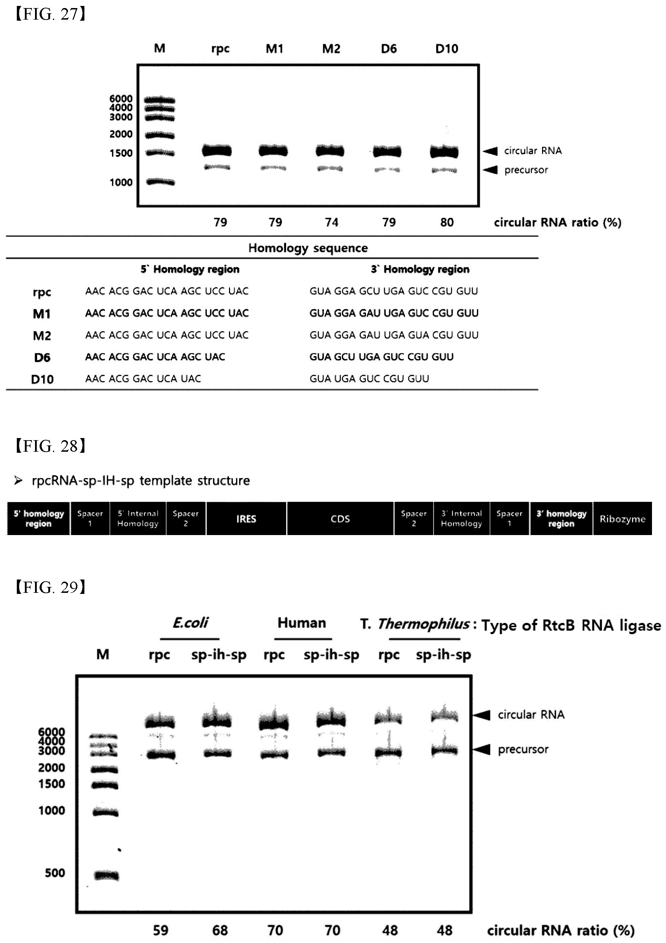

As a result, even if mismatches exist in the 5' homology region and the 3' homology region, there was no effect on the circular RNA synthesis efficiency, and it was confirmed that even when the lengths of the 5' homology region and the 3' homology region were short, there was no effect on the circular RNA synthesis efficiency (FIG. 27).

### Example 8. Circular RNA synthesis efficiency analysis of precursor RNA including additional complementary sequence

A precursor RNA template (rpc-sp-IH-sp template) was designed by adding a 5' internal homology region and a 3' internal homology region complementary to each other to the 3' end of the 5' homology region and the 5' end of the 3' homology region, respectively, in the structure of rpcRNA(rpc-1) of the present invention, and adding spacers to both sides of the internal homology region (FIG. 28). After synthesizing precursor RNA (IRES: Salivirus FHB and CDS: IgG HC) using the template, it was dephosphorylated using a phosphatase, and circular RNA was produced using each of E. coli-derived RtcB RNA ligase, human-derived RtcB RNA ligase, and RtcB RNA ligase derived from *Thermus thermophilus*.

**[Table 4]**

| **sp-IH-sp sequence** | |
|---|---|
| **Spacer 1** | AAAAAACAAAAAACAAAA |
| **5' Internal Homology region** | GCCGGAAACGC AATAGCCG |
| **Spacer 2** | AAAC AAAC AAAC |
| **3' Internal Homology region** | complementary sequence of 5' Internal Homology region |

As a result, in the case of human and *Thermus thermophilus* RtcB, the circular RNA synthesis efficiency provided by the precursor RNA having spacers added to both sides of the internal homology region did not increase; however, in the case of E. coli RtcB, it was confirmed that the precursor RNA had the effect of increasing the circular RNA synthesis efficiency (FIG. 29).

### Example 9. Expression analysis of circular RNA

### 9-1. in vitro expression analysis

Circular RNA comprising Salivirus FHB IRES and a light chain or a heavy chain of IgG as a CDS was synthesized by the method of the present invention or a method using RNA ligase 2. In the case of the method using RNA ligase 2, precursor RNA was synthesized using an EZ-MEGA T7 transcription kit (Enzynomics, EZ039S), circular RNA was synthesized using NEB T4 RNA Ligase 2 (Cat#. M043L), and then dephosphorylation was performed using Antarctic phosphatase (NEB, M026L). Specifically, 1 µg of linearized IVT template, 2 µl of MEGA T7 enzyme Mix, 2 µl of 10x MEGA T7 reaction buffer, 2 µl of 100 mM rATP, 2 µl of 100 mM rCTP, 2 µl of 100 mM rGTP, 2 µl of 100 mM rUTP, and 1.5 µl of 400 mM GMP were added to a PCR strip-tube (Axygen, PCR-0208-CP-C), and the mixture was adjusted to a total volume of 20 µl (GMP:GTP = 3:1). Thereafter, the mixture was reacted at 37°C for 2 hours using a SimpliAmp Thermal Cycler (Thermo). After adding 2 µl of DNase I to the IVT mixture and mixing, an additional reaction was performed at 37°C for 15 minutes. After the additional reaction, 80 µl of NFW was added, and the synthesized precursor RNA was purified according to the manufacturer's manual using an RNeasy Mini Kit (Qiagen, 74106). Then, 75 pmol of the purified precursor RNA, 10 µl of 10x T4 RNA Ligase 2 reaction buffer, and 37.5 U of RNA ligase 2 were added to a PCR strip-tube (Axygen, PCR-0208-CP-C), and the mixture was adjusted to a total volume of 100 µl. The mixture was reacted at 25°C for 18 hours (O/N) using a SimpliAmp Thermal Cycler (Thermo). After the reaction, 80 µl of NFW was added, and the synthesized circular RNA was purified according to the manufacturer's manual using an RNeasy Mini Kit (Qiagen, 74106). Subsequently, 50 µg of the ligated circular RNA, 10 µl of 10x Antarctic phosphatase buffer, and 50 units of Antarctic phosphatase were added, and the mixture was adjusted to a total volume of 100 µl. The mixture was reacted at 37°C for 1 hour and 30 minutes, and the dephosphorylated circular RNA was purified using 7.5 M LiCl. Specifically, LiCl corresponding to 0.5 times the volume of the reaction solution was added to the reaction solution and mixed, left at -20°C for 30 minutes, and then the mixture was centrifuged at 14,000 rpm for 20 minutes. The supernatant was removed, 700 µl of 70% EtOH was added, the mixture was inverted, and then centrifuged at 14,000 rpm for 20 minutes. After centrifugation, the supernatant was removed, and residual 70% EtOH was removed by airdrying to obtain an RNA pellet. The obtained RNA pellet was dissolved in nuclease-free water. The purified dephosphorylated circular RNA was loaded onto a 2% E-Gel EX to confirm the band. The circular RNAs including the light chain and the heavy chain, synthesized by the method of the present invention and the method using RNA ligase 2, respectively, were mixed at a ratio of 1:1 (molar ratio) and introduced into 293T cells using Lipofectamine 3000. Thereafter, the expression of the antibody, which is the target protein, was confirmed by Western blot analysis using an 8% SDS-PAGE gel.

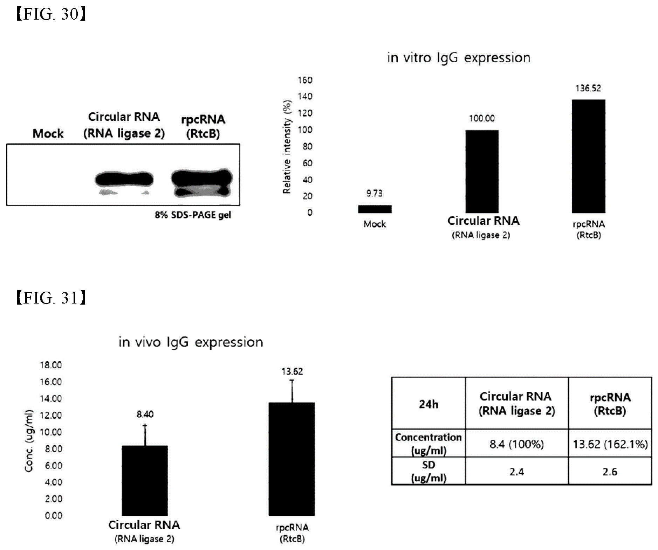

As a result, it was confirmed that the circular RNA synthesized by the two methods effectively generated antibodies. In particular, it was confirmed that when synthesized by the method of the present invention, about 1.4 times more antibodies were produced than by the method using RNA ligase2 (FIG. 30).

### 9-2. in vivo expression analysis

Circular RNAs comprising Salivirus FHB IRES and the light chain or the heavy chain of IgG as CDS were synthesized by the method of the present invention and the method using RNA ligase2, respectively. The circular RNAs comprising the light chain and the circular RNAs comprising the heavy chain were mixed at a ratio of 1:1 (molar ratio) and formulated into LNP (lipid nanoparticle). After intravenous injection of the prepared LNP into mice at a concentration of 0.5 mg/kg, the concentration of blood antibody was measured at 24 hours post-injection.

As a result, in both cases of the two methods, a sufficient amount of antibody was detected in mouse blood (FIG. 31), confirming that antibodies were sufficiently well expressed from the circular RNA administered to the mice. In particular, circular RNA synthesized by the method of the present invention showed about 1.6 times higher concentration of blood antibody than circular RNA synthesized by the RNA ligase2 method (FIG. 31).

## Claims

1. A circular RNA precursor,
wherein the precursor has a structure of 5'-5' homology region-gene of interest (GOI)-3' homology region-ribozyme-3', and the 5' homology region comprises a sequence that is the reverse complement of the 3' homology region.

2. The circular RNA precursor according to claim 1, wherein the 5' homology region further comprises a GGX sequence at the 5' end, and the X is A (adenine), U (uracil), C (cytosine), or (guanine).

3. The circular RNA precursor according to claim 1, wherein the 5' homology region comprises a sequence selected from the group consisting of SEQ ID NOs: 1 to 4.

4. The circular RNA precursor according to claim 1, wherein a triphosphate (P-P-P) is present at the 5' end.

5. The circular RNA precursor according to claim 1, wherein the gene of interest comprises an IRES (internal ribosome entry site) region at the 5' end.

6. The circular RNA precursor according to claim 5, wherein the IRES is an entire sequence, a partial sequence, or a variant of one or more virus-derived sequences selected from the group consisting of Taura syndrome virus, Triatoma virus, Theiler's encephalomyelitis virus, Simian Virus 40, Solenopsis invicta virus 1, Rhopalosiphum padi virus, Reticuloendotheliosis virus, Human poliovirus 1, Plautia stali intestine virus, Kashmir bee virus, Human rhinovirus 2, Homalodisca coagulata virus-1, Human Immunodeficiency Virus type 1, Homalodisca coagulata virus-1, Himetobi P virus, Hepatitis C virus, Hepatitis A virus, Hepatitis GB virus, Foot and mouth disease virus, Human enterovirus 71, Equine rhinitis virus, Ectropis obliqua picorna-like virus, Encephalomyocarditis virus, Drosophila C Virus, Human coxsackievirus B3, Cru- cifer tobamovirus, Cricket paralysis virus, Bovine viral diarrhea virus 1, Black Queen Cell Virus, Aphid lethal paralysis virus, Avian encephalomyelitis virus, Acute bee paralysis virus, Hibiscus chlorotic ringspot virus, Classical swine fever virus, Human FGF2, Human SFTPA1, Human AML1/RUNX1, Drosophila antennapedia, Human AQP4, Human ATIR, Human BAG-1, Human BCL2, Human BiP, Human c-IAP1, Human c-myc, Human eIF4G, Mouse NDST4L, Human LEF1, Mouse HIF1 alpha, Human n.myc, Mouse Gtx, Human p27kip1, Human PDGF2/c-sis, Human p53, Human Pim-1, Mouse Rbm3, Drosophila reaper, Canine Scamper, Drosophila Ubx, Human UNR, Mouse UtrA, Human VEGF-A, Human XIAP, Drosophila hairless, S. cerevisiae TFIID, S. cerevisiae YAP1, tobacco etch virus, turnip crinkle virus, EMCV-A, EMCV-B, EMCV-Bf, EMCV-Cf, EMCV pEC9, Picobirnavirus, HCV QC64, Human Cosavirus E/D, Human Cosavirus F, Human Cosavirus JMY, Rhinovirus NAT001, HRV14, HRV89, HRVC- 02, HRV-A21, Salivirus A SH1, Salivirus FHB, Salivirus NG-J1, Human Parechovirus 1, Crohivirus B, Ye-3, Rosa- virus M-7, Shanbavirus A, Pasivirus A, Pasivirus A 2, Echovirus E14, Human Parechovirus 5, Aichi Virus, Hepatitis A Virus HA16, Phopivirus, CVA10, Enterovirus C, Enterovirus D, Enterovirus J, Human Pegivirus 2, GBV-C GT110, GBV-C K1737, GBV-C Iowa, Pegivirus A 1220, Pasivirus A 3, Sapelovirus, Rosavirus B, Bakunsa Virus, Tremovirus A, Swine Pasivirus 1, PLV-CHN, Pasivirus A, Sicinivirus, Hepacivirus K, Hepacivirus A, BVDV1, Border Disease Virus, BVDV2, CSFV-PK15C, SF573 Dicistrovirus, Hubei Picoma-like Virus, CRPV, Salivirus A BN5, Salivirus A BN2, Salivirus A 02394-01, Salivirus A GUT, Salivirus A CH, Salivirus A SZ1, Salivirus FHB, Salivirus NG-J1, Salivirus A YZ01, Salivirus A YZ02, Salivirus A YZ03, Salivirus A FUAN-01, Salivirus sp isolate ETH, Salivirus A Hu/Baja-5332, Salivirus sp isolate CHN/XJ/env, CVB3, CVB1, Echovirus 7, CVBS, EVA71, CVA3, CVA12, and EV24.

7. The circular RNA precursor according to claim 5, wherein the IRES is an aptamer for elF4G.

8. The circular RNA precursor according to claim 1, further comprising a 5' internal homology region in a 3' direction of the 5' homology region, and a 3' internal homology region in a 5' direction of the 3' homology region, wherein the 5' internal homology region comprises a reverse complement sequence to the 3' internal homology region.

9. The circular RNA precursor according to claim 8, wherein the 5' internal homology region comprises the nucleotide sequence of SEQ ID NO: 10.

10. The circular RNA precursor according to claim 8, further comprising a spacer region in a 5' direction and a 3' direction of the internal homology region.

11. The circular RNA precursor according to claim 10, wherein the spacer comprises a nucleotide sequence of SEQ ID NO: 11 or 12.

12. The circular RNA precursor according to claim 1, wherein the ribozyme is Twister, Twister Sister, Hatchet, Pistol, Hammerhead, Hairpin, Hepatitis Delta Virus (HDV), Neurospora Varkud Satellite (VS), Vg1, glucosamine-6-phosphate synthase (glmS), an engineered synthetic ribozyme, or variants thereof.

13. The circular RNA precursor according to claim 1, further comprising a label.

14. The circular RNA precursor according to claim 13, wherein the label comprises a sequence of PolyA, PolyG, PolyU, or PolyC.

15. The circular RNA precursor according to claim 1, having a size of 250 to 10,000 nt.

16. A recombinant nucleic acid vector for generating the circular RNA precursor of claim 1.

17. The recombinant nucleic acid vector according to claim 16, comprising an operably linked promoter and a gene encoding the circular RNA precursor.

18. A method for producing circular RNA, comprising:
1) a step of synthesizing a precursor RNA by in vitro transcription (IVT) of the recombinant vector of claim 16;
2) a step of treating the synthesized precursor RNA with a phosphatase; and
3) a step of treating the precursor RNA with an RtcB RNA ligase.

19. The method for producing circular RNA according to claim 18, wherein the phosphatase and RtcB RNA ligase are treated simultaneously or sequentially.

20. The method for producing circular RNA according to claim 18, comprising producing circular RNA with a size of 200 to 9950 nt.

21. The method for producing circular RNA according to claim 18, wherein the RtcB RNA ligase is derived from bacteria or mammals.

22. The method for producing circular RNA according to claim 18, comprising further treating with Archease in the step of treating with an RtcB RNA ligase.

23. The method for producing circular RNA according to claim 18, further comprising a step of treating with an exoribonuclease after step 3).

24. Use of a circular RNA precursor for the production of circular RNA.
